# EUROPEAN PATENT APPLICATION

(11) **EP 2 208 712 A1**
(43) Date of publication of application: **21.07.2010**
(21) Application number: 09177526.2
(22) Date of filing: 30.11.2009
(51) Int. Cl.: C02F 3/28, C02F 11/04

(54) **Plant and process for producing biogas**

(30) Priority: 09.12.2008 IT MI20082168
(71) Applicant: Massai Giordano S.R.L., 58100 Grosseto (IT)
(72) Inventor: Massai, Alessandro, 58100 Grosseto (IT); Massai, Micol, 58100 Grosseto (IT)
(74) Representative: Bottero, Carlo

(57) **Abstract**

The present invention relates to a process and a plant for producing biogas, comprising a hydrolytic phase, an acidogenic-acetogenic phase and a methanogenic phase, carried out in separate reactors and under controlled pH, temperature and stirring rate conditions, wherein the hydrolytic phase is carried out under aerobic conditions, preferably with oxygen insufflation.

In an embodiment, the acetogenic phase is maintained at pH ≥ 6, preferably at pH of 6.0 to 7.0, more preferably 6.2 to 6.5.

In another embodiment, each reactor of the plant comprises a coating with net and inert substances arranged along the reactor walls and/or a heating system, preferably a heating coil, arranged along the internal wall of each reactor and at a distance therefrom.

## Description

The present invention relates to a plant and a process for producing biogas.

### STATE OF THE ART

The process of anaerobic fermentation has been widely studied. Its industrial application makes use of various technologies, all of which aim at optimizing the fermentative activity of bacteria so as to exploit as much as possible the chemical energy contained in the organic matrices subjected to fermentation and produce as much biogas as possible.

The fermentative process consists of transformations requiring the intervention of a group of bacteria including several families, each of them being efficient under precise operating conditions (e.g. temperature and pH).

As a matter of fact, optional anaerobic bacteria, which are active during hydrolysis, as well as acetogenic and methanogenic bacteria, which are obligate anaerobic, take part to the fermentation phases.

The currently most widespread plants for anaerobic fermentation are equipped with one digester in which the three main operations of the process occur: hydrolysis of organic compounds, acidogenic-acetogenic digestion as well as methanogenic digestion. This solution is indubitably extremely simple but unfavorable because it is necessary to choose operating conditions that are compatible with the needs of the various families of bacteria co-existing and cooperating in the process.

Since - as was said - every family requires suitable temperature and pH conditions, has its own growth rate requiring a minimum residence time, and is more or less sensitive to the toxicity of given substances, plants and processes for producing biogas were developed in which the various fermentation phases take place in different vessels.

An example of such plants and processes is disclosed in patent application EP1473279. This application describes a plant and a process for the anaerobic production of biogas, wherein anaerobic digestion consists of three phases: a phase of anaerobic hydrolysis of cellulose, sugars and amino acids; an anaerobic acid phase in which simple organic acids (e.g. acetic acids) and alcohols (e.g. ethyl alcohol) are formed; an anaerobic methanogenic phase in which acids and alcohols are turned into methane and carbon dioxide thanks to methanogenic bacteria.

The three phases are carried out in separate reactors and under controlled temperature and pH conditions.

A comminuting phase for the vegetable mass takes place before the hydrolysis phase.

The division into several reactors has the advantage of enabling a better control of pH and reaction conditions.

In the patent referred to above the hydrolysis phase takes place under anaerobic conditions like the acidogenic and methanogenic phases. Since the bacteria used for the hydrolytic phase are facultative anaerobic, the use of a plant in which also the hydrolysis reactor is insulated from the outer environment involves additional costs with respect to a plant in which the acidogenic and methanogenic phases only are kept under anaerobic conditions.

Moreover, in patent EP1473279 the acidogenic phase is carried out at a pH of 2 to 5, in particular 3 to 4, whereas the methanogenic reaction occurs at pH 7-8. The reaction mixture is sent from the acidogenic phase to the methanogenic phase by extravasation, thus introducing a mixture having an extremely acid pH into a reaction mixture with a basic pH. This involves a rapid and substantial change of reaction conditions, which ultimately affects biogas production yield.

Moreover, if the acidogenic phase is carried out at such an acid pH as the one suggested by the patent now discussed, this means that methanogenic bacteria will not be able to develop, not even partially, in the reactor in which the acidogenic phase occurs since these bacteria do not survive at such an acid pH.

As a consequence, the start-up of the methanogenic phase cannot take place during the acidogenic phase, thus resulting again in a lower biogas production yield.

Another disadvantage of the known plant is that for heating the reactors external heat exchangers are used, through which the reaction mixture is led so that it heats up.

In order to implement such a heating system the mixture must be highly liquid so as to flow in the exchangers. In other words, the reaction mixture must be kept at a low concentration of organic material so as to be extremely fluid; a smaller amount of organic material means a lower biogas production and thus a lower reaction yield.

The present invention relates to a plant and a process for producing biogas in which, the hydrolytic, acetogenic and methanogenic phases are carried out in separate reactors.

In particular, an aim of the present invention is to provide a process and a plant for producing biogas starting from vegetable raw material and/or organic sewage or starting from organic sewage only, which is an alternative to known plants and processes.

The process and plant according to the invention produce biogas under a better and more effective control of reaction conditions, in particular pH and temperature, than known plants. This results in an equivalent or even higher biogas production yield with respect to known plants.

The technical task and the aims referred to above are basically achieved by a plant and a process for producing biogas, comprising the technical features disclosed in one or more of the appended claims.

The present invention relates to a plant and a process for producing biogas comprising a hydrolytic-acidogenic phase, an acidogenic-acetogenic phase and a methanogenic phase, which phases are carried out in separate reactors and under controlled pH, temperature and stirring rate conditions, the hydrolytic phase takes place under aerobic conditions. In the plant according to the invention, the reactor in which the hydrolysis reaction takes place operates under aerobic conditions.

In a preferred embodiment of the invention, the hydrolytic phase takes place under aerobic conditions with oxygen insufflation. The plant according to the invention comprises a reactor in which the hydrolytic phase is carried out, which reactor is equipped with an oxygen insufflation device.

In an embodiment of the process and plant according to the invention, the acetogenic phase is maintained at a pH ≥ 6. In other words, the acetogenic bioreactor is equipped with a pH-control device and, optionally, with a device for delivering a pH-adjusting solution, which is able to maintain the reaction mixture at a pH of 6 or higher.

In another embodiment, the walls of the reactors are coated inside with synthetic net and inert substances. This coating highly promotes the concentration and proliferation of resident bacteria.

Resident bacteria are different from floating bacteria in that the latter are suspended in the reaction mixture and travel from one reactor to the other together with the mixture. Resident bacteria are those which remain anchored to reactor walls and/or to the coating thereof. The larger the amount of resident bacteria, the longer the contact time between the reaction mixture and the microorganisms responsible for the reaction, and thus the higher the reaction yield.

In another embodiment, the reactors of the plant are heated by means of a heating system, preferably heating coils, arranged along the internal walls at a suitable distance from the latter in order to enable a perimetric movement of the reaction mixture between the heating system and the wall, so as to allow the mixture to get in deep contact with resident bacteria. The reaction mixture thus executes perimetric movements adding to the central stirring obtained by means of a conventional mechanical stirrer.

The perimetric movements (i.e. the shift of the mixture from the reactor wall and the heating system) added to the central stirring, which keeps the reaction mixture under constant stirring, enable the reaction mixture to get in deep contact with resident bacteria, thus causing a higher reaction yield.

In a preferred embodiment, the walls of the reactors are coated with synthetic net and inert substances and heated by means of a heating system, preferably heating coils, arranged along the internal walls at a given distance from the latter.

The heating system is arranged at a distance ranging from 20 to 70 cm from the internal wall of the reactor, and at a distance from the coating ranging from 5 to 70 cm.

The process according to the invention advantageously comprises a hydrolysis reaction mixture, an acetogenic reaction mixture and a methanogenic reaction mixture, placed in separate reactors, subjected to a central stirring, obtained by means of a mechanical stirrer, and to a perimetric stirring, adding to the central stirring and obtained by arranging along the internal wall of each reactor and a distance therefrom a heating system, preferably heating coils.

Thanks to the heating system for the reactors as described above, the reaction mixture needs not be kept very fluid, as in known plants, since it must not be pumped into external heat exchangers. The concentration of organic material in the reaction mixture is therefore higher than in mixtures of the prior art, which positively affects biogas production yield.

In the plant and process according to the invention, the hydrolytic phase is preceded by a raw material comminuting phase under controlled temperature and pH. The plant and the process also include, after the methanogenic phase, one or more phases involving purification and recovery/recycling of water and of undigested vegetable material.

Further characteristics and advantages of the present invention will be more readily apparent from the indicative and thus non-limiting description of a preferred, though not exclusive embodiment of a plant and a process, as shown in the accompanying drawings, in which:
- Figure 1 is a flow chart showing an embodiment of a plant according to the present invention, in which the raw material feeding the plant is vegetable material, optionally mixed with organic sewage;
- Figure 2 is a flow chart showing an embodiment of a plant according to the present invention, in which the raw material feeding the plant is organic sewage.

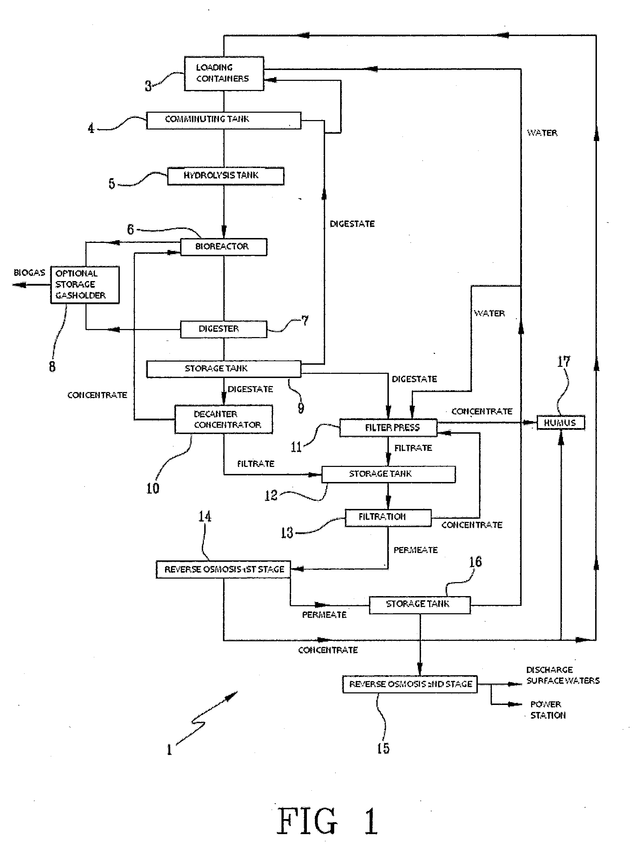

With reference to Figure 1, the numeral 1 globally refers to the plant for producing biogas fed with vegetable raw material (biomass) or with a mixture of vegetable material and organic sewage.

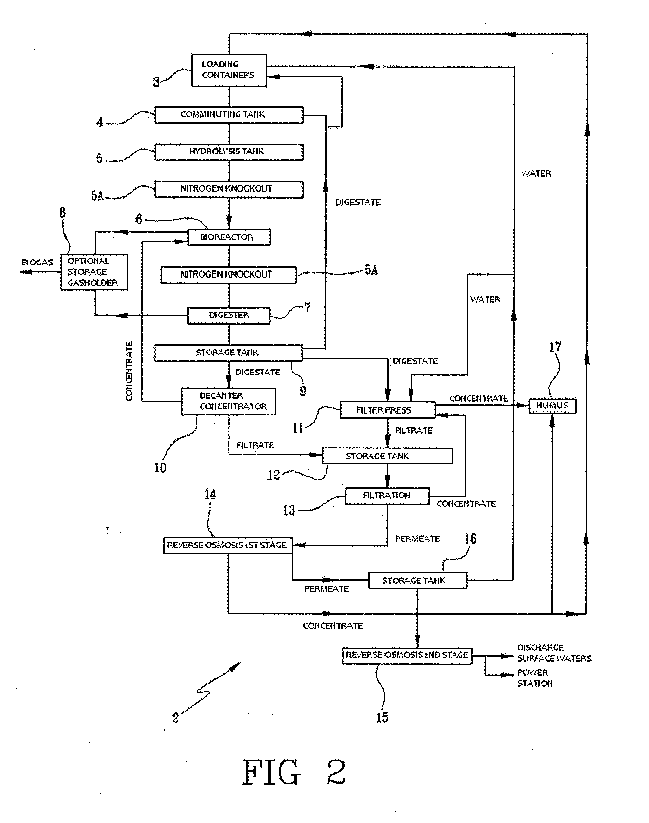

With reference to Figure 2, the numeral 2 globally refers to the biogas production plant fed with organic waste only.

Vegetable raw material or vegetable biomass refers to products, by-products, crop residues and waste. Example of plant residues are straw, maize stalks, leaves, mowing residues; example of agro-alimentary residues are tomatoes, leguminous vegetables, cereals, fruits and vegetables, beets, grape pomace and olive residues; examples of vegetable crops are maize, hemp, sorghum, sunflower, rapeseed and lucerne. Other examples of vegetable biomass are market waste, waste from processing industries, algae and the solid fraction of municipal solid waste.

Organic sewage refers to e.g. zootechnical sewage such as slurry, manure (from cows, pigs, horses), poultry droppings, rumen, civil sludge, organic sludge, oil, fat, blood, fleshings, etc.

With reference to Figures 1 and 2, the solid vegetable biomass is loaded into the loading container 3 where it is added with hot water, advantageously recycled from reverse osmosis treatment, and/or organic animal sewage and/or with osmotic concentrate from reverse osmosis treatment (described below), so as to obtain a suspension with the desired concentration in organic dry matter (ODM).

The addition of organic sewage and/or osmotic concentrate to the vegetable biomass is carried out in order to compensate the intrinsic nitrogen lack of such raw material.

The loading container is insulated and enables to keep an operating temperature in the range from 0°C to 30°C.

There are preferably two or more loading containers 3 operating in parallel and preferably under aerobic conditions.

From the loading container 3 the biomass added with water and/or organic sewage and/or osmotic concentrate is sent to the comminuting tank 4, preferably to two or more comminuting tanks arranged in parallel.

The loading containers and the comminuting tanks are arranged in series so as to let the amount of biomass required for feeding the plant, which will be subjected to continuous comminuting and mixing, cyclically flow in.

During this pre-treatment the biomass is heated so as to make it easier to achieve the temperature required in the first hydrolytic phase. The duration of this pre-treatment phase, divided into loading, comminuting and pre-heating and unloading, depends on the biomass type, on the duration of the following hydrolytic phase and on tank size. Advantageously, the residence time of the biomass in the comminuting tanks is of 12 to 24 hours, up to a maximum of 48 hours when the biomass has a high fat content. Temperatures of 0°C to 34°C are applied.

The comminuting tanks 4 operate under aerobic conditions.

In the comminuting tanks the biomass is preferably added with a part of the digested matter getting out of the digester 7 and coming from the optional storage tank 9 arranged downstream from the digester.

In these tanks the biomass is turned from a chopped mass with a length of few centimeters to a pulp-like texture.

After the residence time mentioned above, the biomass gets from the comminuting tanks 4 to the hydrolysis tank 5 by means of a pumping system or by extravasation. The hydrolysis tank 5 advantageously comprises 2 to 8 hydrolysis tanks, preferably 4 to 8 tanks arranged in series. The hydrolysis tanks operate under aerobic conditions. The tanks are insulated and equipped with a heating system and optionally with an oxygenation system.

The volume of insufflated oxygen depends on the biomass amount and composition. The amount of oxygen introduced into the hydrolytic tanks must be such as to partially oxidize the biomass though enabling at the same time the development of an amount of bacteria useful to start acid digestion. Also the undigested or partially undigested biomass from the digester can thus be completely digested. As a result, reaction yield is increased.

In the hydrolysis tanks, due to the presence of extracellular enzymes, inoculated and/or produced by hydrolyzing bacteria, the complex organic molecules of the biomass are turned into monomers (fat acids, glycerol, alcohols, amino acids, simple sugars).

All the bacteria operating in this phase are facultative anaerobic, some of them being able only to produce the enzymes for hydrolyzing organic matrices, others being able also to metabolize monomers produced by hydrolysis so as to form acetic acid, short-chain organic acids, branched-chain alcohols with more than one carbon atom, aromatic fat acids, etc. The bacteria that are present in this phase are of the type *Lactobacillus* (e.g. *Lactobacillus casei, Lactobacillus plantarum*) and of the type *Streptococcus* (e.g. *Streptococcus lactii, Streptococcus cemoris*).

The residence time of the biomass inside the hydrolysis tank depends on the nature of the latter and is divided into loading, heating and mixing, hydrolysis and discharge. The duration of the loading phase is the same as the duration of the discharge phase from the comminuting tanks. The duration of the heating phase depends on the temperature at which the biomass enters; during heating the biomass will still be mixed thanks to the use of submersed vertical pumps or horizontal stirrers.

Hydrolysis starts when a given temperature is achieved, ranging from 25°C to 40°C, and lasts from 12 to 48 hours depending on biomass type and on tank size. The discharge into the following tank, i.e. into the acetogenic bioreactor 6, carried out by means of a pumping system or by extravasation, is performed in small hourly doses so as to ensure a minimum thermal and biochemical shock of the recipient environment. For instance, 1/24 of the daily hourly feeding of the biomass can be discharged into the bioreactor.

The pH of the hydrolytic phase is maintained in a range of 4.0 to 10.0, preferably 6.0 to 8.0, more preferably 6.0 to 7.5.

During these first phases the bacterial-enzymatic mixtures will be optionally inoculated, advantageously by recycling a part of the digested sludge getting out of the digester in the comminuting tanks.

The hydrolyzed biomass is sent to an ammonia extractor 5A (Figure 2) by means of a pumping system or by extravasation. The ammonia extractor or other equivalent system for drastically reducing excess nitrogen (e.g. a filtration system) can be arranged between the hydrolysis tanks 5 and the bioreactor 6, between the bioreactor 6 and the digester 7, or in both positions; it is present in plants according to Figure 2 only, wherein the feeding raw material is rich in salts and ammoniacal and/or protein nitrogen, i.e. in the case of organic sewage.

As a result of the metabolic processes performed by acidogenic bacteria, all the nitrogen contained in the matrix, except for the part anabolized by bacteria, is turned into ammoniacal nitrogen which, if present as NH₃ at concentrations of 1200-1300 ppm, is toxic and harmful in particular for methanogenic microorganisms active in the downstream digester where pH increase due to the progressive transformation of acetate ions into methane results in the shift of the balance NH₃-NH₄⁺ towards the formation of free ammonia.

This would involve a "poisoning" of methanogenic fermentation and a decrease in production yield.

The ammonia extractor 5A or other equivalent system for drastically reducing excess nitrogen is not used in plants fed with vegetable biomass only; however, it can be provided for those plants fed with mixtures of vegetable biomass and organic sewage, if the concentration of organic sewage is so high to cause the development of an inadmissible nitrogen amount for methanogenic fermentation.

An example of alternative system for drastically reducing nitrogen is a membrane filtration system or a low pressure filtration enabling to separate the biomass getting out of the hydrolysis tanks or of the bioreactor into two streams: a microfiltered stream without suspended bodies and free of organic molecules, which contains most of the nitrogen and of the dissolved salts to be sent to reverse osmosis (connection not shown in the figure); the other stream, containing all the solid material (mass of bacteria and insoluble substances), all the organic material (alcohols, aromatic acids, branched-chain organic molecules, etc.) and part of the solution, is sent to the bioreactor, to the digester or to both.

This operation thus enables to produce biogas also from organic matrices which otherwise it would be impossible to use as exclusive feeding for the plant because of too low C/N ratios or due to the high salt content.

The hydrolyzed biomass, which has optionally got through the system for drastically reducing nitrogen, is sent to the bioreactor 6 by means of a pumping system or by extravasation. The bioreactor is fed not only with the biomass from the hydrolysis tanks, but also with the biomass from the decanter/concentrator 10 and/or with the undigested or partially digested biomass from the digester 7. The undigested biomass is thus recycled, which contributes to increase process yield.

The bioreactor comprises one or more insulated and heated tanks. Each tank is preferably equipped with such a stirring system as to keep the whole biomass in motion and to mix it maintaining a biomass speed of 1.80 to 3.00 m/min, never above 3.50 m/min.

Mixing occurs with an intermittance calculated as a function of the chemical and physical composition of the biomass, of its viscosity and related electric conductivity. Each tank is equipped with a heating system for maintaining an internal temperature of 30°C to 50°C, a system for controlling temperature in one or more places inside the tank, of the redox reaction, conductivity and pH. The pH must be ≥ 6, preferably of 6.0 to 7.0, more preferably of 6.2 to 6.5.

The pH must be kept at 6 or above since acid pHs do not cause any development of methanogenic bacteria operating in syntrophy with acetogenic bacteria (as will be explained in further detail below) and causing a start-up of the reaction involving methane production. As is shown in the figures, biogas production already starts in the bioreactor where biogas is taken, together with the biogas from the digester, optionally stored in a storage gasholder 8 and eventually sent to the power station.

The start-up of the methanogenic reaction in the bioreactor results in a higher biogas production yield. Moreover, since the pH of the acetogenic phase is 6 or higher and the pH of the methanogenic phase is of 7-8, the travel of the reaction mixture from the bioreactor to the digester involves no huge changes in pH or reaction conditions in general. More controlled reaction conditions allow higher reaction yields.

In order to prevent the pH from sinking below 6, a system for introducing an acidity regulator into the bioreactor can be provided.

In an embodiment of the plant according to the invention, the wall of the bioreactor is coated inside with a synthetic net for retaining inert substances.

This type of coating allows the bacteria contained in the reaction mixture to colonize better.

In this way is possible to increase the concentration of resident bacteria with respect to floating bacteria present in the reaction mixture.

The increase in concentration of resident bacteria allows an increase in process efficiency and therefore in biogas production yield.

Tank size depends on the nature of the biomass fed and on the residence time varying from 2 to 15 days, preferably from 5 to 12 days.

The bacteria contained in the bioreactor and involved in the acetogenic phase are: acetogens, which turn into acetates, hydrogen and carbon dioxide the metabolites produced by acidogenic bacteria; homoacetogens, which produce acetate ion by reacting carbon dioxide and hydrogen; hydrogenotrophic methanogenic microorganisms, which produce methane using carbon dioxide and hydrogen dissolved in the mixture containing the biomass.

All these operations take place inside the bioreactor under anaerobic conditions and under such operating conditions of temperature, pH, load by mass and load by volume, stirring rate as to be compatible with the characteristics of the bacteria operating therein.

Also a partial recirculation of the biomass getting out of methanogenic digesters, whose aim is to keep a high bacteria concentration and to enable a suitable residence time for such bacteria, reaches the bioreactor in addition to the stream from the hydrolysis phase.

This allows to: reduce the bioreactor volume; act effectively in the transformation of propionate and butyrate ions into acetate ions, reducing the risk that volatile fat acids flow through methanogenic digesters in such an amount as to be toxic for methane-producing microorganisms; extend the residence time of bacteria in the digesters and thus reduce sludge formation, with the double advantage of minimizing the problem concerning the disposal thereof and of reducing the amount of COD (chemical oxygen demand) used by bacteria to self-reproduction purposes (and therefore removed from methane production).

Some fermentation products such as fat acids with a chain having more than two carbon atoms, alcohols with a chain having more than one carbon atom, branched-chain products and aromatic fat acids, cannot be used directly in methanogenesis. In the acetogenesis process these products are oxidized to acetates and hydrogen by obligated hydrogen-producing bacteria, which operate in syntrophy with hydrogenotrophic methanogenic bacteria. Keeping in mind that the efficiency of the stream of hydrogen between producers and consumers is inversely proportional to their mutual distance, the maximum efficiency is achieved when obligated hydrogen-producing bacteria and hydrogenotrophic methanogens are joined together. Acetogenic bacteria make use not only of the syntrophic action of hydrogenotrophic methanogens but also of the syntrophy with acetoclastic methanogens since these remove acetate ions formed as a result of the oxidation reaction of volatile fat acids and help the development thereof. In order to promote syntrophy and thus an effective metabolism of the various bacterial families, it is necessary that during the acetogenic step bacteria granulation, i.e. the formation of sheet-like granules with methanogenic bacteria in the middle thereof, then acetogens and eventually acidogens, is favored even with the addition of granules of inert substances.

If, thanks to the characteristics of the organic matrix which is fed, it is not necessary to arrange an ammonia extractor or similar system for drastically reducing nitrogen or salts, the stream getting out of the hydrolytic phase is sent directly to the acetogenic digester.

If conversely extraction is necessary for controlling nitrogen and/or salts, the concentrate from extraction only gets into the acetogenic digester, where it is added with utility water in such an amount as to bring bacterial concentration, salt content, and nitrogen concentration to desired values.

Though considering that during this phase together with acetogenic bacteria also methanogenic bacteria (both hydrogenotrophic and acetoclastic) with which they must work in syntrophy are already present though in smaller amounts, the operating conditions (pH, temperature, mixing rate, retention time, etc.) are chosen so as to optimize the acetogenic activity and thus minimize the concentration of short-chain organic acids in the reaction mixture to be sent to the actual methanogenic phase.

The reaction mixture getting from the acetogenic to the methanogenic step contains all of the acetate ions and all of the hydrogen which the few methanogenic bacteria present in that step succeeded in methanizing.

The production of particularly methane-rich biogas occurs precisely in this phase. In order to promote methanation it is necessary to provide a correct retention time and a bacteria age enabling the development thereof and allowing a strong formation of bacterial granules.

In order to achieve a good methane yield it is fundamental that formed bacterial granules are sufficiently wetted by the surrounding reaction mixture so as to take the required nutrients from such a mixture. Since a large amount of insoluble gas (methane) is formed, which gas might drag granules towards the surface of the liquid (balloon effect, typical of flotation), it is necessary that gas development occurs through microbubbles.

It is also important that the liquid mixing rate is such as not to prevent or disturb the formation of new granules and to maintain at the same time a homogeneous concentration of metabolites in the whole liquid mass of the digester. In order to favor such conditions, also the internal wall of the digester (as for the bioreactor) is coated with net and inert substances so as to increase the concentration of resident bacteria.

With respect to the acetogenic bioreactor, during the methanogenesis the pH tends to increase (however, under stable process conditions it must be approximately neutral). In order to increase the kinetics of biochemical reactions, if there is no risk of an excess concentration of free NH₃, it may be useful to raise temperature of few degrees, which would also enable to sink surface tension and improve the wettability of bacterial granules.

In order to control NH₃ concentration when treating nitrogen-rich matrices, it is envisaged to send to the digester a biogas stream so that CO₂ contained therein reacts with the ammonium ion so as to form the buffer NH₄HCO₃. Considering that methanogenic microorganisms have longer doubling time than acidogens and acetogens, water retention time and residence time for bacteria in this phase are longer than in the previous phase.

The digester 7 comprises one or more insulated and heated tanks, fed with the biomass from the bioreactor/s 6.

Each tank will be equipped with such a stirring system as to keep the whole biomass under motion and mix it successfully maintaining a biomass speed of 1.80 to 3.00 m/min, never above 3.50 m/min.

Mixing occurs with an intermittance calculated as a function of the chemical and physical composition of the biomass, of its viscosity and related electric conductivity.

Each tank is equipped with a heating system for maintaining an internal temperature of 40°C to 58°C, a system for controlling temperature in one or more places inside the tank (advantageously in the middle and near the wall), the oxydation degree, conductivity and pH, whose range is of 7 to 8, preferably of 6.8 to 7. Preferably, the pH never sinks below 6.5 even when using an inoculation system for an optional acidity regulator.

A synthetic net for retaining insert substances is applied onto the walls of the digester and coats a strip of variable height along the whole circumference of the tank.

Tank size depends on the type of feeding biomass and on the residence time which may range from 15 to 30 days.

The digested biomass is then conveyed into such an anaerobic concentration system 10 as to eliminate a part of exceeding water and thus increase the percentage of suspended organic substance without damaging the bacteria contained therein.

Before being sent to the decanter/concentrator 10, the biomass may optionally be stored in a storage tank 9 placed downstream from the digester. In this case, after storage the biomass is partially sent to the decanter/concentrator 10, partially recycled to the comminuting tank 4 and partially sent to the filter press 11 where the solid-liquid separation occurs. The more concentrated solid part is mixed with the concentrate from reverse osmosis so as to obtain the humus 17 which is used as improver/fertilizer for agricultural soil.

Filter pressing enables to obtain panels (cakes) with 45-50% of dry solid matter, containing the bacterial biomass formed during the process and the inert solids. These retain as precipitate almost all of the phosphor entered with feeding, and small amounts of potassium and nitrogen dissolved in the reaction mixture which said panels are imbibed with. These cakes, mixed with the concentrate from reverse osmosis, are very good soil improvers which thanks to their optimal water retention properties (up to three times their own weight) improve soil ability to retain water, and which thanks to their good chelation and ion exchange properties reduce the danger of heavy metals present in the water circulating in the soil trickling into water sheets.

The liquid part getting out of the filter press (filtrate), which contains soluble salts contained in the feeding and still has 0.3-0.5% of suspended solid bodies, is optionally,sent to a storage tank 12 arranged downstream from the filter press, then to the filtration system 13, preferably a microfiltration system, and then to the reverse osmosis system 14, 15.

The filtration system 13 separates the more concentrated solid part, which is recirculated to the filter press 11, from the liquid part, which is sent to reverse osmosis 14, 15.

A part of the bacterial mass concentrated in the decanter/concentrator 10 is recirculated by introducing it into the bioreactor 6; the amounts to be recirculated are calculated on the basis on the level of bacterial mass and suspended mass to be maintained inside the tanks so as to balance the digestion process. A part of the biomass from the decanter/concentrator 10 is sent to the storage tank 12.

The part of biomass stored in the storage tank 12 can be used as such in agriculture as soil fertilizer and improver, or it can be treated with the filtration system 13 so as to obtain a solid-liquid separation, in which the concentrated solid part is sent to the filter press 11 and the liquid part is subjected to reverse osmosis 14, 15.

The biogas produced in the previous phases is optionally conveyed through the storage gasholder 8, where it undergoes a first cooling process and a chemical treatment for drastically reducing hydrogen sulphide.

The chemical treatment is based on the insufflation of a precise amount of oxygen so as to react the acid contained in the gas and precipitate it also thanks to the action of specific bacteria.

The following are subjected to reverse osmosis 14, 15: the liquid stream rich in nitrogen and dissolved salts, coming from the nitrogen extractor or other system for separating excess nitrogen 5A optionally arranged downstream from the hydrolysis tanks 5 and/or between the bioreactor 6 and the digester 7; the liquid stream from the decanter/concentrator 10, previously filtered; the filtrate from the filter press 11, previously filtered.

The reverse osmosis system comprises a first filter 14 from which a permeate and a concentrate get out.

Almost only water gets through the permeate, which water is wholly or partly recycled or used to other industrial purposes or discharged into surface water-courses; soluble salts and ammoniacal nitrogen remain in the concentrate.

Therefore, the permeate is partially recirculated to the loading containers 3 and partially sent to the second osmosis filter 15, optionally after being stored in the storage tank 16.

The choice of the treatment for this concentrate can aim at eliminating nitrogen or at recovering it both to agronomic purposes and so as to be returned to the plant for improving a process parameter (C/N ratio).

In the first case there are two possibilities: a) catalytic removal of ammoniacal nitrogen turned into simple nitrogen, which can be discharged into the atmosphere; b) transformation of ammoniacal nitrogen into simple nitrogen by means of known biochemical treatments of nitrification-denitrification or nitritation-denitritation.

In the second case, the purpose of nitrogen recovery can be: a) agronomic use: the concentrate, rich in ammoniacal nitrogen, is saturated under cold conditions with carbon dioxide contained in the biogas in a suitable small carbonatation tower (not shown).

Nitrogen is thus fixed as ammonium carbonate, which remains dissolved in the concentrated solution. This solution is then absorbed by the cakes obtained from the filter press, which if necessary are added with straw or other organic mineral substances with absorbing properties, so as to obtain a soil improver for agriculture (28-30% of total solid organic dry matter) rich in fertilizers (humus, 17).

Compared with other methods the proposed solution has two advantages: a) ammonia is stabilized as ammonium carbonate, a salt which is stable up to about 60°C even in solid state; b) the use of biogas itself as a fixer for ammonia avoids the use of other chemical reagents (e.g. sulphuric acid), thus avoiding related dangers and costs.

## Claims

1. A process for producing biogas including a hydrolytic phase, an acidogenic-acetogenic phase and a methanogenic phase, carried out in separate reactors and under controlled pH, temperature and stirring rate conditions, wherein the hydrolytic phase is carried out under aerobic conditions.

2. A process according to claim 1, wherein the hydrolytic phase is carried out under aerobic conditions with oxygen insufflation.

3. A process according to claim 1 or 2, wherein the acetogenic phase is maintained at a pH ≥ 6.

4. A process according to any one of the claims 1 to 3, wherein said acetogenic phase is carried out at a pH from 6.0 to 7.0, preferably from 6.2 to 6.5.

5. A process according to any one of the claims 1 to 4, wherein said hydrolytic phase, said acetogenic phase and said methanogenic phase are subjected to a central stirring, obtained by a mechanical stirrer, and to a perimetric stirring, which is added to the central stirring, obtained through the positioning, along the internal wall of each reactor and spaced therefrom, of a heating system, preferably a heating coil.

6. A process according to claim 5, wherein along the internal wall of each reactor a net for she containment of inert substances is positioned.

7. A plant for producing biogas, operating according to the process of any one of the claims 1 to 6, including at least a hydrolysis reactor (5), at least an acetogenic reactor (6) connected in series with the hydrolysis reactor and at least a methanogenic reactor (7) connected in series with the acetogenic reactor, wherein said hydrolysis reactor operates under aerobic conditions.

8. A plant according to claim 7, wherein said hydrolysis reactor operates under aerobic conditions and is equipped with an oxygen insufflation device.

9. A plant according to claim 7 or 8, wherein said acetogenic reactor is equipped with a pH-control device and, optionally, a device for delivering a pH-adjusting solution, capable of maintaining the pH of the reaction mixture at values equal or higher than 6, preferably at a pH from 6.0 to 7.0, more preferably from 6.2 to 6.5.

10. A plant according to any one of the claims 7 to 9, wherein each reactor includes a net and inert substances coating placed along the internal wall of each reactor and/or a heating system, preferably a heating coil, placed along the internal wall of each reactor and spaced therefrom.

11. A plant according to claim 10, said heating system being placed at a distance, from the wall of each reactor, between 20 and 70 cm and at a distance from the coating between 5 and 70 cm.

12. A plant according to any one of the claims 7 to 11 comprising a mechanical stirrer placed in proximity of the central zone of at least one reactor.
